# NOUVEAU FASCICULE DE BREVET EUROPEEN

(11) **EP 2 111 848 B2**
(45) Date de publication et mention de la décision concernant l'opposition: **27.03.2019**
(45) Mention de la délivrance du brevet: 30.03.2016
(21) Numéro de dépôt: 09158016.7
(22) Date de dépôt: 16.04.2009
(51) Int. Cl.: A61K 8/36, A61K 8/365, A61K 8/41, A61K 8/58, A61Q 5/12

(54) **Composition cosmétique comprenant au moins un composé organique de silicium, au moins un tensioactif cationique et au moins un acide organique, et un procédé de traitement cosmétique mettant en oeuvre ladite composition**
Kosmetische Zusammensetzung, die mindestens eine organische Siliziumverbindung, mindestens ein kationisches Tensid und mindestens eine organische Säure umfasst, sowie kosmetisches Behandlungsverfahren, bei dem diese Zusammensetzung zum Einsatz kommt
Cosmetic composition comprising at least one organic silicon compound, at least one cationic surface-active agent and at least one organic acid, and cosmetic treatment method using said composition

(30) Priorité: 25.04.2008 FR 0852794
(43) Date de publication de la demande: 28.10.2009
(73) Titulaire: L'Oréal, 75008 Paris (FR)
(72) Inventeur: Woodland, Frédéric, 75018, Paris (FR); Lazzeri, Pascale, 13540 Puyricard (FR)
(74) Mandataire: Dossmann, Gérard

(56) Documents cités:
- EP-A2- 0 992 528
- WO-A-01/22925
- US-A1- 2006 280 716
- KAZUYUKI YAHAGI, NAOKO HOSHINO, HAJIME HIROTA: "Solution behaviour of new cationic surfactants derived from Guerbet alcohols and their use in hair conditioners", INTERNATIONAL JOURNAL OF COSMETIC SCIENCE, no. 13, 1991, pages 221-234, XP002514907,
- ANONYME: "Auswahl pflegender Wirkstoffe für einen Haarschaum", SÖFW-JOURNAL, vol. 131, no. 3, 2005, pages 38-43, XP002514908,

## Description

La présente invention concerne une composition cosmétique pour le traitement des fibres kératiniques, en particulier des fibres kératiniques humaines telles que les cheveux, comprenant, dans un milieu cosmétiquement acceptable, un ou plusieurs composés organiques du silicium, un ou plusieurs tensioactifs cationiques et un ou plusieurs acides organiques. La présente invention concerne également un procédé de traitement cosmétique des fibres kératiniques ainsi qu'une utilisation mettant en oeuvre ladite composition cosmétique.

Les cheveux sont généralement abîmés et fragilisés par l'action des agents atmosphériques extérieurs tels que la lumière et les intempéries, et par des traitements mécaniques ou chimiques tels que le brossage, le peignage, les teintures, les décolorations, les permanentes et/ou les défrisages.

Ainsi, pour remédier à ces inconvénients, il est maintenant usuel d'avoir recours à des soins capillaires impliquant la mise en oeuvre de compositions de soins qui permettent de conditionner les cheveux à la suite de ces traitements afin de leur conférer notamment de la brillance, de la douceur, de la souplesse, de la légèreté, un toucher naturel ainsi que des propriétés de démêlage.

Ces compositions de soins capillaires peuvent être par exemple des shampooings conditionneurs ou des après-shampoings pouvant se présenter sous la forme de gels, de lotions capillaires ou de crèmes plus ou moins épaisses.

Par ailleurs, on a constaté que les consommateurs sont de plus en plus à la recherche de compositions de soins qui permettent non seulement de conditionner les cheveux de manière convenable mais également de procurer des effets coiffants satisfaisants.

En particulier, les personnes ayant des cheveux fins ou bouclés sont généralement à la recherche de produits de soins qui procurent des effets coiffants apportant de la masse, du corps et du volume aux cheveux fins et du dessin aux boucles des cheveux frisés.

Toutefois, les compositions de soins usuelles procurent des effets coiffants qui sont relativement faibles et irréguliers, notamment en termes de dessins de boucle et de volume.

En effet, il est connu que l'introduction de composés organiques cosmétiquement actifs tels que des polymères cationiques et des silicones dans des compositions de soins telles que des après-shampooings, permet de conférer aux cheveux des propriétés de démêlage, de souplesse et de légèreté. Cependant, les propriétés coiffantes qui sont apportées aux cheveux restent encore nettement insuffisantes.

Il existe donc un réel besoin de mettre au point des compositions cosmétiques qui permettent de conditionner les cheveux de manière satisfaisante tout en apportant des effets coiffants puissants, notamment en termes de masse, de corps, de volume et de dessin aux boucles des cheveux.

La demanderesse a découvert, de façon surprenante, qu'il était possible de formuler des compositions pour le traitement cosmétique des fibres kératiniques, ayant les propriétés recherchées, comprenant un ou plusieurs composés organiques du silicium tels que définis ci-après, un ou plusieurs tensioactifs cationiques et un ou plusieurs acides organiques.

En effet, il a été constaté que l'ajout de composés organiques du silicium dans des compositions comprenant un ou plusieurs tensioactifs cationiques et un ou plusieurs acides organiques permettait de conduire à un gainage satisfaisant des cheveux permettant de conférer aux cheveux un toucher doux satisfaisant.

De plus, les compositions selon l'invention confèrent des effets coiffants puissants, notamment en apportant de la masse, du corps et du volume aux cheveux.

Par ailleurs, les compositions selon l'invention permettent de faciliter la mise en forme des cheveux, en particulier des cheveux fins.

Enfin, les compositions selon l'invention permettent également de conférer des effets coiffants aux cheveux bouclés, notamment en terme de dessin et de contrôle des boucles.

La présente invention concerne notamment une composition cosmétique pour le traitement des fibres kératiniques, en particulier des fibres kératiniques humaines telles que les cheveux, comprenant dans un milieu cosmétiquement acceptable :
- un ou plusieurs composés organiques du silicium choisis parmi les silanes de formule (III) telle que définie ci-après;
- un ou plusieurs tensioactifs cationiques ; et
- un ou plusieurs acides organiques différents des tensioactifs.

La présente invention concerne également un procédé de traitement cosmétique des cheveux, comprenant l'application sur lesdites fibres de la composition selon l'invention.

Elle concerne aussi l'utilisation de la composition selon l'invention comme shampoing ou conditionneur.

D'autres objets et caractéristiques, aspects et avantages de l'invention apparaîtront encore plus clairement à la lecture de la description et des exemples qui suivent.

Le ou les composés organiques du silicium utilisés dans la composition selon invention sont des organosilanes choisis parmi les composés de formule (III) : 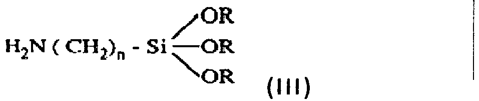 dans laquelle les radicaux R, identiques ou différents, sont choisis parmi les radicaux alkyle en C1-C6, de préférence en C1-C2, et n est un nombre entier de 1 à 6, de préférence de 2 à 4.

De préférence, les silanes sont solubles dans l'eau et encore plus préférentiellement solubles à la concentration de 2%, mieux à la concentration de 5% et encore mieux à la concentration de 10% en poids dans l'eau à la température de 25°C±5°C et à la pression atmosphérique. Par soluble, on entend la formation d'une phase macroscopique unique.

De façon particulièrement préférée, le composé organique du silicium présent dans la composition selon l'invention est le 3-aminopropyl triéthoxysilane.

Le ou les composés organiques du silicium peuvent être présents dans la composition selon l'invention dans une teneur allant de 0,1 à 20 % en poids, de préférence dans une teneur en poids allant de 1 à 15 % en poids, et plus préférentiellement dans une teneur en poids allant de 2,5 à 12% en poids, par rapport au poids total de la composition.

La composition selon la présente invention contient un ou plusieurs tensioactifs cationiques, de préférence monomériques.

A titre d'exemples de tensioactif cationique, on peut notamment citer les sels d'amines grasses primaires, secondaires ou tertiaires, éventuellement polyoxyalkylénées et les sels d'ammonium quaternaire éventuellement polyoxyalkylénés.

De préférence, les tensioactifs cationiques sont choisis parmi les sels d'ammonium quaternaire éventuellement polyoxyalkylénés.

A titre de sels d'ammonium quaternaires, on peut notamment citer, par exemple :
- ceux qui présentent la formule générale (IV) suivante : 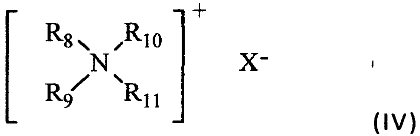 dans laquelle les radicaux R8 à R11, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes.
   Les radicaux aliphatiques sont par exemple choisis parmi les radicaux alkyle, alcoxy, polyoxyalkylène (C2 -C6), alkylamide, alkyl(C12 -C22)amidoalkyle(C2 -C6), alkyl(C12 -C22 )acétate, hydroxyalkyle, comportant environ de 1 à 30 atomes de carbone ; X est un anion choisi dans le groupe des halogénures, phosphates, acétates, lactates, alkyl(C2 -C6)sulfates, alkyl- ou alkylaryl-sulfonates ;
- les sels d'ammonium quaternaire de l'imidazoline, comme par exemple ceux de formule (V) suivante : 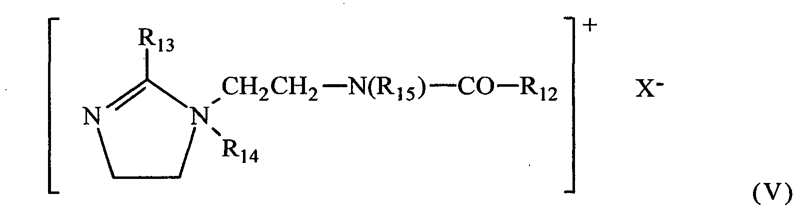 dans laquelle R12 représente un radical alcényle ou alkyle comportant de 8 à 30 atomes de carbone, par exemple dérivés des acides gras du suif, R13 représente un atome d'hydrogène, un radical alkyle en C1-C4 ou un radical alcényle ou alkyle comportant de 8 à 30 atomes de carbone, R14 représente un radical alkyle en C1-C4, R15 représente un atome d'hydrogène, un radical alkyle en C1 -C4 , X⁻ est un anion choisi dans le groupe des halogénures, phosphates, acétates, lactates, alkylsulfates, alkyl- ou alkylaryl-sulfonates. De préférence, R12 et R13 désignent un mélange de radicaux alcényle ou alkyle comportant de 12 à 21 atomes de carbone, par exemple dérivés des acides gras du suif, R14 désigne un radical méthyle, R15 désigne un atome d'hydrogène. Un tel produit est par exemple commercialisé sous la dénomination REWOQUAT^{®} W 75 par la société REWO ;
- les sels de diammonium quaternaire de formule (VI) : 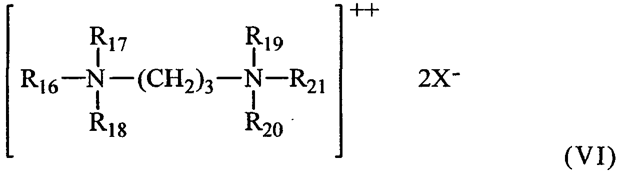 dans laquelle R16 désigne un radical aliphatique comportant environ de 16 à 30 atomes de carbone, R17 , R18 , R19 , R20 et R21 , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propane suif diammonium ;
- les sels d'ammonium quaternaire contenant au moins une fonction ester, tels que ceux de formule (VII) suivante : 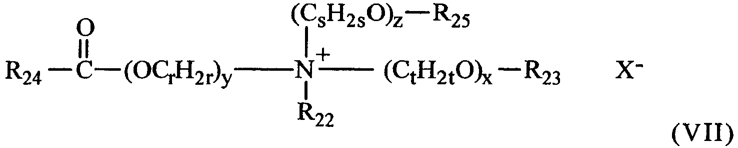 dans laquelle :
   R22 est choisi parmi les radicaux alkyles en C1 -C6 et les radicaux hydroxyalkyles ou dihydroxyalkyles en C1 -C6 ;
   R23 est choisi parmi :
      - le radical 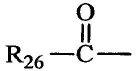
      - les radicaux R27 hydrocarbonés en C1-C22, linéaires ou ramifiés, saturés ou insaturés,
      - l'atome d'hydrogène,
   R25 est choisi parmi :
      - le radical 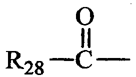
      - les radicaux R29 hydrocarbonés en C1-C6, linéaires ou ramifiés, saturés ou insaturés,
      - l'atome d'hydrogène,
   R24, R26 et R28, identiques ou différents, sont choisis parmi les radicaux hydrocarbonés en C7-C21, linéaires ou ramifiés, saturés ou insaturés;
   r, s et t, identiques ou différents, sont des entiers valant de 2 à 6 ;
   y est un entier valant de 1 à 10 ;
   x et z, identiques ou différents, sont des entiers valant de 0 à 10 ;
   X- est un anion simple ou complexe, organique ou inorganique;
   sous réserve que la somme x + y + z vaut de 1 à 15, que lorsque x vaut 0 alors R23 désigne R27 et que lorsque z vaut 0 alors R25 désigne R29.

Les radicaux alkyles R22 peuvent être linéaires ou ramifiés et plus particulièrement linéaires.

De préférence R22 désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R23 est un radical R27 hydrocarboné, il peut être long et avoir de 12 à 22 atomes de carbone, ou court et avoir de 1 à 3 atomes de carbone.

Lorsque R25 est un radical R29 hydrocarboné, il a de préférence 1 à 3 atomes de carbone.

Avantageusement, R24, R26 et R28, identiques ou différents, sont choisis parmi les radicaux hydrocarbonés en C11-C21, linéaires ou ramifiés, saturés ou insaturés, et plus particulièrement parmi les radicaux alkyle et alcényle en C11-C21, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X- est encore plus particulièrement le chlorure ou le méthylsulfate.

On utilise plus particulièrement dans la composition selon l'invention, les sels d'ammonium de formule (VII) dans laquelle :
- R22 désigne un radical méthyle ou éthyle,
- x et y sont égaux à 1 ;
- z est égal à 0 ou 1 ;
- r, s et t sont égaux à 2 ;
- R23 est choisi parmi :
   - le radical 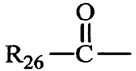
- les radicaux méthyle, éthyle ou hydrocarbonés en C14-C22,
- l'atome d'hydrogène ;
- R25 est choisi parmi :
   - le radical 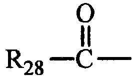
   - l'atome d'hydrogène ;
   - R24, R26 et R28 , identiques ou différents, sont choisis parmi les radicaux hydrocarbonés en C13-C17 , linéaires ou ramifiés, saturés ou insaturés, et de préférence parmi les radicaux alkyles et alcényles en C13-C17 , linéaires ou ramifiés, saturés ou insaturés.

Avantageusement, les radicaux hydrocarbonés sont linéaires.

On peut citer par exemple les composés de formule (VII) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxy éthyl-méthylammonium, de monoacyloxyéthyl-hydroxyéthyl-diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthane sulfonate de méthyle, le paratoluènesulfonate de méthyle, la chlorhydrine du glycol ou du glycérol.

De tels composés sont par exemple commercialisés sous les dénominations DEHYQUART<^{®}>par la société HENKEL, STEPANQUAT^{®} par la société STEPAN, NOXAMIUM<^{®}>par la société CECA, REWOQUAT^{®}WE 18 par la société REWO-WITCO.

La composition selon l'invention contient de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthyl sulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (IV), on préfère d'une part, les chlorures de tétraalkylammonium comme, par exemple, les chlorures de dialkyldiméthylammonium ou d'alkyltriméthylammonium ou alkylaralkylediméthylammonium dans lesquels le radical alkyle comporte environ de 12 à 22 atomes de carbone, en particulier les chlorures de béhényltriméthyl ammonium, de distéaryldiméthylammonium, de cétyltriméthylammonium, de benzyldiméthylstéarylammonium ou encore, d'autre part, le chlorure de palmitamidopropyltriméthylammonium ou le chlorure de stéaramidopropyldiméthyl-(myristylacétate)-ammonium commercialisé sous la dénomination CERAPHYL<^{®}>70 par la société VAN DYK.

De préférence, le tensioactif cationique utilisé dans la composition selon l'invention est choisi parmi les chlorures de béhényltriméthyl ammonium et de cétyltriméthyl ammonium et leurs mélanges.

Le ou les tensioactifs cationiques utilisés dans la composition selon la présente invention peuvent être présents dans la composition dans une quantité variant de 0,1 à 6% en poids, de préférence en une quantité variant de 0,5 à 3% en poids.

Par acide organique, on entend tout composé organique non polymérique comportant une ou plusieurs fonctions acides choisies parmi les fonctions acide carboxylique, acide sulfonique, acide phosphorique.

L'acide organique n'est pas un tensioactif.

Encore plus préférentiellement, le poids moléculaire de l'acide organique est inférieur à 250, mieux inférieur à 200.

Les acides organiques peuvent être des acides aminés.

Le ou les acides organiques sont choisis de préférence parmi l'acide acétique, l'acide propanoïque, l'acide butanoïque, l'acide lactique, l'acide malique, l'acide glycolique, l'acide ascorbique, l'acide maléïque, l'acide phtalique, l'acide succinique, la taurine, l'acide tartrique, l'arginine, la glycine, l'acide glucuronique, l'acide gluconique et l'acide citrique.

Encore plus préférentiellement, les acides organiques selon l'invention sont les acides carboxyliques.

Encore plus préférentiellement encore, l'acide organique utilisé dans la composition selon l'invention est l'acide acétique, l'acide citrique et de préférence l'acide lactique.

Dans la composition, l'acide organique peut être sous forme libre ou salifiée.

Le ou les acides organiques utilisés dans la composition selon la présente invention peuvent être présents en une teneur exprimée en acides libres allant de 0,1 à 10% en poids, de préférence en une teneur allant de 0,5 à 8% en poids, et encore plus préférentiellement en une teneur allant de 1 à 5% en poids, par rapport au poids total de la composition.

La composition selon la présente invention peut comprendre également un ou plusieurs tensioactifs additionnels choisis parmi les tensioactifs anioniques, amphotères et non ioniques.

Les tensioactifs anioniques pouvant être utilisés dans les compositions de l'invention sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C6-24 et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides (alkyl en C6-24)éther-carboxyliques polyoxyalkylénés, les acides (alkyl en C6-24)(aryl en C6-24)éther-carboxyliques polyoxyalkylénés, les acides (alkyl en C6-24)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et leurs mélanges, en particulier sous forme de sels de métaux alcalins ou alcalino-terreux, d'ammonium, d'amine ou d'aminoalcool.

Lorsqu'ils sont présents, la quantité du ou des tensioactifs anioniques est de préférence comprise dans l'intervalle allant de 0,1 à 4 % en poids, par rapport au poids total de la composition.

Encore plus préférentiellement, la composition ne contient pas de tensioactifs anioniques.

Des exemples de tensioactifs non-ioniques additionnels utilisables dans les compositions de la présente invention sont décrits par exemple dans " Handbook of Surfactants" par M.R. PORTER, éditions Blackie & Son (Glasgow and London), 1991, pp 116-178 . Ils sont choisis notamment parmi les alcools, les alpha-diols, les alkyl(C1-20)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitan éthoxylés ayant de 2 à 30 motifs d'oxyde d'éthylène, les esters d'acides gras du saccharose, les esters d'acides gras du polyéthylèneglycol, les (alkyl en C6-24)polyglycosides, les dérivés de N-(alkyl en C6-24)glucamine, les oxydes d'amines tels que les oxydes d'(alkyl en C10-14)amines ou les oxydes de N-(acyl en C10-14)-aminopropylmorpholine.

Lorsqu'ils sont présents, la quantité du ou des tensioactifs non ioniques additionnels est de préférence comprise dans l'intervalle allant de 0,01 à 20% en poids, mieux encore de 0,1 à 10 % en poids par rapport au poids total de la composition.

Les agents tensioactifs amphotères ou zwittérioniques, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C8-20)bétaïnes, les sulfobétaïnes, les (alkyl en C8-20)amido(alkyl en C6-8)bétaïnes ou les (alkyl en C8-20)amido(alkyl en C6-8)sulfobétaïnes.

Parmi les dérivés d'amines, on peut citer les produits commercialisés sous la dénomination MIRANOL<^{®}>, tels que décrits dans les brevets US 2 528 378 et US 2 781 354 et classés dans le dictionnaire CTFA, 3<ème>édition, 1982, sous les dénominations Amphocarboxy-glycinate et Amphocarboxypropionate de structures respectives (A) et (B) :

Ra-CONHCH2CH2-N(Rb)(Rc)(CH2COO<->) (A)

dans laquelle :
Ra représente un groupe alkyle dérivé d'un acide Ra -COOH présent dans l'huile de coprah hydrolysée, un groupe heptyle, nonyle ou undécyle,
Rb représente un groupe bêta-hydroxyéthyle, et
Rc représente un groupe carboxyméthyle ; et

Ra'-CONHCH2CH2-N(B)(B') (B)

dans laquelle :
B représente -CH2 CH2 OX',
B' représente -(CH2)z -Y', avec z = 1 ou 2,
X' représente le groupe -CH2 CH2 -COOH ou un atome d'hydrogène,
Y' représente -COOH ou le groupe -CH2 -CHOH-SO3 H,
Ra ' représente un groupe alkyle d'un acide Ra '-COOH présent dans l'huile de coprah ou dans l'huile de lin hydrolysée, un groupe alkyle, notamment en C17 et sa forme iso, un groupe en C17 insaturé.

Ces composés sont classés dans le dictionnaire CTFA, 5<ème>édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®}C2M concentré.

Parmi les tensioactifs amphotères ou zwittérioniques cités ci-dessus, on utilise de préférence les (alkyl en C8-20)-bétaïnes, les (alkyl en C8-20)-amido(alkyl en C6-8)bétaïnes et leurs mélanges.

Lorsqu'ils sont présents, la quantité du ou des tensioactifs amphotères ou zwittérioniques est de préférence comprise dans l'intervalle allant de 0,01 à 20 %, mieux encore de 0,1 à 10 % en poids par rapport au poids total de la composition.

Par milieu cosmétiquement acceptable, on entend un milieu compatible avec les fibres kératiniques, telles que les cheveux.

Le milieu cosmétiquement acceptable est constitué d'eau ou d'un mélange d'eau et d'un ou plusieurs solvants cosmétiquement acceptables choisis parmi les alcools inférieurs en C1-C4, tels que l'éthanol, l'isopropanol, le tertio-butanol ou le n-butanol ; les polyols tels que le glycérol, le propylèneglycol et les polyéthylèneglycols ; et leurs mélanges.

Le pH des compositions selon l'invention varie généralement de 3 à 11 et de préférence de 7 à 10.

La composition selon l'invention peut comprendre en outre un ou plusieurs additifs classiques bien connus dans la technique, tels que; des épaississants ou régulateurs de viscosité, naturels ou synthétiques ; des alcools gras en C12-C30; des céramides ; des esters gras huileux tels que le myristate d'isopropyle, le myristate de myristyle, le palmitate de cétyle et le stéarate de stéaryle ; des huiles minérales, végétales ou synthétiques telles que les α-oléfines ou l'huile de palme ; des vitamines ou provitamines ; des polymères cationiques ou amphotères ; des agents de stabilisation du pH, des conservateurs ; et des colorants.

Le ou les agents épaississants peuvent être choisis parmi les agents épaississants cellulosiques, par exemple l'hydroxyéthylcellulose, l'hydroxypropylcellulose et le carboxyméthylcellulose, la gomme de guar et ses dérivés, par exemple l'hydroxypropyl guar, commercialisé par la société RHODIA sous la référence JAGUAR HP 105, les gommes d'origine microbienne, telle que la gomme de xanthane et la gomme de scléroglucane, les agents épaississants synthétiques tels que les homopolymères réticulés d'acide acrylique ou d'acide acrylamidopropanesulfonique, par exemple le Carbomer, les polymères associatifs non ioniques, anioniques, cationiques ou amphotères, tels que les polymères commercialisés sous les appellations PEMULEN TR1 ou TR2 par la société GOODRICH, SALCARE SC90 par la société CIBA, ACULYN 22, 28, 33, 44 ou 46 par la société ROHM & HAAS et ELFACOS T210 et T212 par la société AKZO.

De préférence, les additifs sont choisis parmi les polymères cationiques et les agents épaississants, notamment les polymères associatifs non ioniques tels que les polyuréthanes commercialisés sous les appellations ACULYN 44 et ACULYN 46.

L'homme de métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

Les compositions conformes à l'invention peuvent être utilisées pour le conditionnement des fibres kératiniques, en particulier des cheveux, par exemple comme après-shampooings.

De préférence, les compositions de l'invention sont des après shampooings à rincer ou non.

Un autre objet de l'invention est un procédé de traitement cosmétique des fibres kératiniques, telles que les cheveux, qui consiste à appliquer une quantité efficace d'une composition telle que décrite ci-dessus, sur lesdites fibres, éventuellement à rincer après un éventuel temps de pose.

L'exemple suivant illustre la présente invention.

### Exemple 1:

On prépare les compositions (A), (B), (C) et (D) à partir des ingrédients indiqués dans le tableau ci-dessous dont les quantités sont exprimées en pour cent en poids en matières actives, par rapport au poids total de la composition.

| Compositions | A (invention) | B (invention) | C (invention) | D (comparatif) |
|---|---|---|---|---|
| 3-aminopropyltriéthoxysilane | 10 | 10 | 10 | - |
| Alcool cétylstéarylique (C16/C18 50/50) | 2,5 | 3 | 5 | 5 |
| Mélange de myristate de myristyle, de palmitate de cétyle et de stéarate de stéaryle | 0,5 | - | - | - |
| Huile de palme | - | 2 | - | - |
| Chlorure de béhényltriméthyl ammonium | 1,2 | - | 2,4 | 2,4 |
| Chlorure de cétyltriméthyl ammonium | - | 0,8 | - | - |
| Acide lactique | 4 | 4 | 0,5 | 0,5 |
| Eau | qsp 100% | qsp 100% | qsp 100% | qsp 100% |

Appliquées en tant qu'après shampooings, les compositions (A) et (B) confèrent aux cheveux de la masse, du volume ainsi qu'un toucher doux satisfaisant.

De plus, ces compositions permettent de faciliter la mise en forme des cheveux fins et d'apporter une meilleure définition de boucle aux cheveux bouclés.

Un essai comparatif entre les compositions (C) et (D) montre que la composition (C) apporte plus de corps à la chevelure que la composition (D).

### Exemple 2:

On prépare les compositions (E), (F), (G) et (H) à partir des ingrédients indiqués dans le tableau ci-dessous dont les quantités sont exprimées en pour cent en poids en matières actives, par rapport au poids total de la composition.

| Compositions | E | F | G | H |
|---|---|---|---|---|
| 3-aminopropyltriéthoxysilane | 10 | 10 | 2 | 2 |
| Alcool Cétylstéarylique (C16/C18 50/50) | 4 | 4,75 | 4 | 4,75 |
| Mélange Myristate/Palmitate/Stéarate de Myristyle/Cétyle/Stéaryle | | 1 | | 1 |
| PEG-150/Alcool stéarylique/Copolymère SMDI (ACULYN 46 de ROHM&HAAS) | 0,3 | 0,375 | 0,3 | 0,375 |
| POLYQUATERNIUM-37 (SALCARE SC 95 de CIBA) | 0,5 | 0,5 | 0,5 | 0,5 |
| Chlorure de béhényltriméthyl ammonium (GENAMIN KDMP de CLARIANT) | 0,4 | | 0,4 | |
| Chlorure de cétyltriméthyl ammonium (DEHYQUART A OR de COGNIS) | | 0,25 | | 0,25 |
| Acide lactique | 3,5 pH 9±0,3 | 3,5 pH 9±0,3 | 0,7 pH 9±0,3 | 0,7 pH 9±0,3 |
| Eau | Qsp 100 | Qsp 100 | Qsp 100 | Qsp 100 |

Appliquées en tant qu'après shampooings, les compositions (E), (F), (G) et (H) confèrent aux cheveux de la masse, du volume ainsi qu'un toucher doux satisfaisant.

De plus, ces compositions permettent de faciliter la mise en forme des cheveux fins et d'apporter une meilleure définition de boucle aux cheveux bouclés.

## Revendications

1. Composition cosmétique pour le traitement des fibres kératiniques, en particulier des fibres kératiniques humaines telles que les cheveux, **caractérisé en ce qu'**elle comprend, dans un milieu cosmétiquement acceptable
- un ou plusieurs composés organiques du silicium de formule (III): 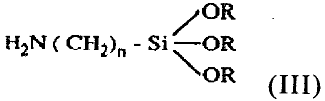 dans laquelle les radicaux R, identiques ou différents, sont choisis parmi les radicaux alkyle en C1-C6 et n est un nombre entier de 1 à 6
- un ou plusieurs tensioactifs cationiques; et
- un ou plusieurs acides organiques différente(s) des tensioactifs.

2. Composition cosmétique **caractérisée en ce que** le composé organique du silicium est le 3-aminopropyltriéthoxysilane.

3. Composition cosmétique selon la revendication 1 ou 2, **caractérisée en ce que** les composés organiques du silicium sont présents dans une teneur allant de 0,1 à 20 % en poids, de préférence dans une teneur en poids allant de 1 à 15 % en poids, et plus préférentiellement dans une teneur en poids allant de 2,5 à 12% en poids, par rapport au poids total de la composition.

4. Composition cosmétique selon l'une quelconque des revendications 1 à 3, **caractérisée en ce que** le ou les tensioactifs cationiques sont choisis parmi les sels d'amines grasses primaires, secondaires ou tertiaires, éventuellement polyoxyalkylénées et les sels d'ammonium quaternaire éventuellement polyoxyalkylénés et leurs mélanges.

5. Composition cosmétique selon la revendication 4, **caractérisée en ce que** les sels d'ammonium quaternaire sont choisis parmi:
- ceux qui présentent la formule générale (IV) suivante : 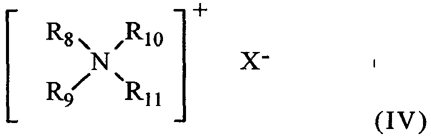 dans laquelle les radicaux R8 à R11, qui peuvent être identiques ou différents, représentent un radical aliphatique, linéaire ou ramifié, comportant de 1 à 30 atomes de carbone, ou un radical aromatique tel que aryle ou alkylaryle ; X est un anion choisi dans le groupe des halogénures, phosphates, acétates, lactates, alkyl(C2 -C6)sulfates, alkyl- ou alkylaryl-sulfonates ;
- les sels d'ammonium quaternaire de l'imidazoline,
- les sels de diammonium quaternaire de formule (VI) : 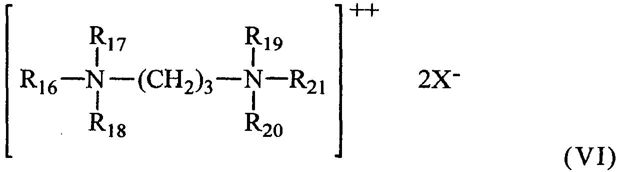 dans laquelle R₁₆ désigne un radical aliphatique comportant environ de 16 à 30 atomes de carbone, R₁₇ , R₁₈, R₁₉, R₂₀ et R₂₁, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates,
- les sels d'ammonium quaternaire contenant au moins une fonction ester.

6. Composition cosmétique selon l'une quelconque des revendications précédentes, **caractérisée en ce que** le ou les tensioactifs cationiques sont présents en une teneur allant de 0,1 à 6% en poids, de préférence en une teneur allant de 0,5 à 3% en poids, par rapport au poids total de la composition.

7. Composition cosmétique selon l'une quelconque des revendications précédentes, **caractérisée en ce que** le ou les acides organiques sont choisis parmi l'acide acétique, l'acide propanoïque, l'acide butanoïque, l'acide lactique, l'acide malique, l'acide glycolique, l'acide ascorbique, l'acide maléïque, l'acide phtalique, l'acide succinique, la taurine, l'acide tartrique, l'arginine, la glycine, l'acide glucuronique, l'acide gluconique et l'acide citrique.

8. Composition cosmétique selon l'une quelconque des revendications précédentes, **caractérisée en ce que** le ou les acides organiques sont présents en une teneur allant de 0,1 à 10% en poids, de préférence en une teneur allant de 0,5 à 8% en poids, et encore plus préférentiellement en une teneur allant de 1 à 5% en poids, par rapport au poids total de la composition.

9. Procédé de traitement cosmétique des fibres kératiniques, **caractérisé en ce que** l'on applique la composition cosmétique telle que définie selon l'une quelconque des revendications précédentes, sur lesdites fibres, et **en ce que** l'on rince après un éventuel temps de pose.

10. Utilisation d'une composition selon l'une quelconque des revendications 1 à 8, comme après-shampooing.

## Patentansprüche

1. Kosmetische Zusammensetzung zur Behandlung von Keratinfasern, insbesondere menschlichen Keratinfasern wie dem Haar, **dadurch gekennzeichnet, dass** sie in einem kosmetisch unbedenklichen Medium Folgendes umfasst:
- eine oder mehrere organische Siliciumverbindungen der Formel (III): 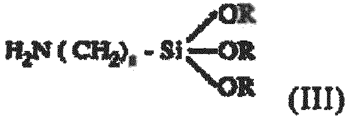 wobei die Reste R, die gleich oder verschieden sind, aus C₁-C₆-Alkylresten ausgewählt sind und n eine ganze Zahl von 1 bis 6 ist,
- ein oder mehrere kationische Tenside und
- eine oder mehrere organische Säuren, die von den Tensiden verschieden sind.

2. Kosmetische Zusammensetzung, **dadurch gekennzeichnet, dass** es sich bei der organischen Siliciumverbindung um 3-Aminopropyltriethoxysilan handelt.

3. Kosmetische Zusammensetzung nach Anspruch 1 oder 2, **dadurch gekennzeichnet, dass** die organischen Siliciumverbindungen in einem Gehalt im Bereich von 0,1 bis 20 Gew.-%, vorzugsweise in einem Gewichtsgehalt im Bereich von 1 bis 15 Gew.-% und weiter bevorzugt in einem Gewichtsgehalt im Bereich von 2,5 bis 12 Gew.-%, bezogen auf das Gesamtgewicht der Zusammensetzung, vorliegen.

4. Kosmetische Zusammensetzung nach einem der Ansprüche 1 bis 3, **dadurch gekennzeichnet, dass** das kationische Tensid bzw. die kationischen Tenside aus Salzen von primären, sekundären oder tertiären Fettaminen, die gegebenenfalls polyoxyalkyleniert sind, quaternären Ammoniumsalzen, die gegebenenfalls polyoxyalkyleniert sind, und Mischungen davon ausgewählt ist bzw. sind.

5. Kosmetische Zusammensetzung nach Anspruch 4, **dadurch gekennzeichnet, dass** die quaternären Ammoniumsalze aus:
- denjenigen der folgenden allgemeinen Formel (IV): 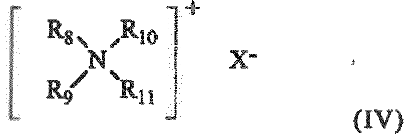 worin die Reste R₈ bis R₁₁, die gleich oder verschieden sein können, für einen linearen oder verzweigten aliphatischen Rest mit 1 bis 30 Kohlenstoffatomen oder einen aromatischen Rest wie Aryl oder Alkylaryl stehen; X für ein Anion aus der Gruppe der Halogenide, Phosphate, Acetate, Lactate, (C₂-C₆)Alkylsulfate, Alkyl- oder Alkylarylsulfonate steht;
- quaternären Ammoniumsalzen von Imidazolin;
- quaternären Diammoniumsalzen der Formel (VI): 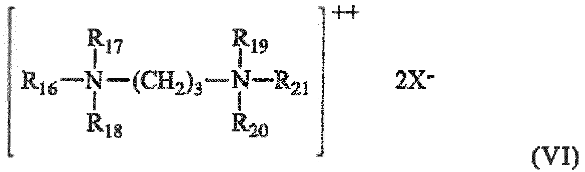 worin R₁₆ für einen aliphatischen Rest mit ungefähr 16 bis 30 Kohlenstoffatomen steht, R₁₇, R₁₈, R₁₉, R₂₀ und R₂₁, die gleich oder verschieden sind, aus Wasserstoff oder einem Alkylrest mit 1 bis 4 Kohlenstoffatomen ausgewählt sind und X für ein Anion aus der Gruppe der Halogenide, Acetate, Phosphate, Nitrate und Methylsulfate steht;
- quaternären Ammoniumsalzen mit mindestens einer Esterfunktion
ausgewählt sind.

6. Kosmetische Zusammensetzung nach einem der vorhergehenden Ansprüche, **dadurch gekennzeichnet, dass** das kationische Tensid bzw. die kationischen Tenside in einem Gehalt im Bereich von 0,1 bis 6 Gew.-%, vorzugsweise in einem Gehalt im Bereich von 0,5 bis 3 Gew.-%, bezogen auf das Gesamtgewicht der Zusammensetzung, vorliegt bzw. vorliegen.

7. Kosmetische Zusammensetzung nach einem der vorhergehenden Ansprüche, **dadurch gekennzeichnet, dass** die organische Säure bzw. die organischen Säuren aus Essigsäure, Propansäure, Butansäure, Milchsäure, Glykolsäure, Ascorbinsäure, Maleinsäure, Phthalsäure, Bernsteinsäure, Taurin, Weinsäure, Arginin, Glycin, Glucuronsäure, Gluconsäure und Citronensäure ausgewählt ist bzw. sind.

8. Kosmetische Zusammensetzung nach einem der vorhergehenden Ansprüche, **dadurch gekennzeichnet, dass** die organische Säure bzw. die organischen Säuren in einem Gehalt im Bereich von 0,1 bis 10 Gew.-%, vorzugsweise in einem Gehalt im Bereich von 0,5 bis 8 Gew.-% und noch weiter bevorzugt in einem Gehalt im Bereich von 1 bis 5 Gew.-%, bezogen auf das Gesamtgewicht der Zusammensetzung, vorliegt bzw. vorliegen.

9. Verfahren zur kosmetischen Behandlung von Keratinfasern, **dadurch gekennzeichnet, dass** man die kosmetische Zusammensetzung gemäß einem der vorhergehenden Ansprüche auf die Fasern aufbringt und nach einer fakultativen Einwirkungszeit ausspült.

10. Verwendung einer Zusammensetzung nach einem der Ansprüche 1 bis 8 als Conditioner.

## Claims

1. Cosmetic composition for treating keratin fibres, in particular human keratin fibres such as the hair, **characterized in that** it comprises, in a cosmetically acceptable medium:
- one or more organosilicon compounds of formula (III): 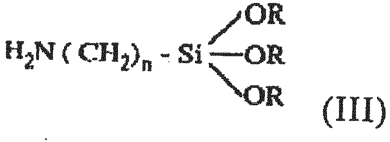 in which the radicals R, which may be identical or different, are chosen from C₁-C₆ alkyl radicals and n is an integer from 1 to 6,
- one or more cationic surfactants; and
- one or more organic acids other than the surfactants.

2. Cosmetic composition, **characterized in that** the organosilicon compound is 3-aminopropyltriethoxysilane.

3. Cosmetic composition according to Claim 1 or 2, **characterized in that** the organosilicon compounds are present in a content ranging from 0.1% to 20% by weight, preferably in a weight content ranging from 1% to 15% by weight and more preferentially in a weight content ranging from 2.5% to 12% by weight, relative to the total weight of the composition.

4. Cosmetic composition according to any one of Claims 1 to 3, **characterized in that** the cationic surfactant(s) are chosen from optionally polyoxyalkylenated primary, secondary or tertiary fatty amine salts and optionally polyoxyalkylenated quaternary ammonium salts, and mixtures thereof.

5. Cosmetic composition according to Claim 4, **characterized in that** the quaternary ammonium salts are chosen from:
- those which have the general formula (IV) below: 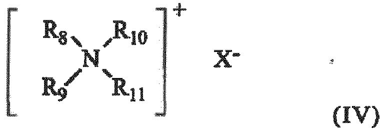 in which the radicals R₈ to R₁₁, which may be identical or different, represent a linear or branched aliphatic radical comprising from 1 to 30 carbon atoms, or an aromatic radical such as aryl or alkylaryl; X is an anion chosen from the group of halides, phosphates, acetates, lactates, (C₂-C₆) alkyl sulfates, alkylsulfonates and alkylarylsulfonates;
- quaternary ammonium salts of imidazoline;
- quaternary diammonium salts of formula (VI): 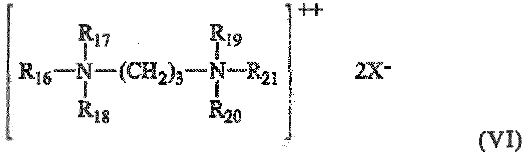 in which R₁₆ denotes an aliphatic radical comprising from about 16 to 30 carbon atoms, R₁₇, R₁₈, R₁₉, R₂₀ and R₂₁, which may be identical or different, are chosen from hydrogen and an alkyl radical comprising from 1 to 4 carbon atoms, and X is an anion chosen from the group of halides, acetates, phosphates, nitrates and methyl sulfates;
- the quaternary ammonium salts containing at least one ester function.

6. Cosmetic composition according to any one of the preceding claims, **characterized in that** the cationic surfactant(s) are present in a content ranging from 0.1% to 6% by weight and preferably in a content ranging from 0.5% to 3% by weight, relative to the total weight of the composition.

7. Cosmetic composition according to any one of the preceding claims, **characterized in that** the organic acid(s) are chosen from acetic acid, propanoic acid, butanoic acid, lactic acid, glycolic acid, ascorbic acid, maleic acid, phthalic acid, succinic acid, taurine, tartaric acid, arginine, glycine, glucuronic acid, gluconic acid and citric acid.

8. Cosmetic composition according to any one of the preceding claims, **characterized in that** the organic acid(s) are present in a content ranging from 0.1% to 10% by weight, preferably in a content ranging from 0.5% to 8% by weight and even more preferentially in a content ranging from 1% to 5% by weight, relative to the total weight of the composition.

9. Cosmetic process for treating keratin fibres, **characterized in that** the cosmetic composition as defined according to any one of the preceding claims is applied to said fibres, and **in that** the composition is rinsed out after an optional leave-on time.

10. Use of a composition according to any one of Claims 1 to 8, as a hair conditioner.
